(19) Europäisches Patentamt
European Patent Office
Office européen des brevets

(11) Publication number: **0 197 706**
**B1**

(12) # EUROPEAN PATENT SPECIFICATION

(45) Date of publication of patent specification: **18.10.89**

(51) Int. Cl.$^4$: **C 07 C 87/60,** C 07 C 85/04, C 07 C 79/12

(21) Application number: **86302226.5**

(22) Date of filing: **26.03.86**

(54) **Synthesis of bromethalin intermediate.**

(30) Priority: **01.04.85 US 718576**

(43) Date of publication of application:
**15.10.86 Bulletin 86/42**

(45) Publication of the grant of the patent:
**18.10.89 Bulletin 89/42**

(84) Designated Contracting States:
**AT BE CH DE FR GB IT LI NL SE**

(56) References cited:
**CH-A- 326 157**
**US-A-4 316 988**

**SYNTHESIS, no. 3, March 1980, page 215, Georg Thieme Verlag, Stuttgart, DE; J.J. KULAGOWSKI et al.: "Improved preparation of 2-nitrodiphenylamines"**

(73) Proprietor: **ELI LILLY AND COMPANY**
**Lilly Corporate Center**
**Indianapolis Indiana 46285 (US)**

(72) Inventor: **Biggs, William Scott**
**22 Glen Drive**
**Mooresville, IN 46158 (US)**
Inventor: **Rieder, Brent Jeffrey**
**510 N. State Street**
**Greenfield, IN 46140 (US)**

(74) Representative: **Hudson, Christopher Mark et al**
**Erl Wood Manor**
**Windlesham Surrey GU20 6PH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 197 706 B1

**Description**

N-Alkyldiphenylamines represent an important class of biologically active compounds. In particular, the N-methyldiphenylamine 2,4,6-tribromo-N-methyl-2′,4′-dinitro-6′-trifluoromethyldiphenylamine, generically known as bromethalin, is a potent rodenticide. However, it has been determined that the desmethyl derivative of bromethalin, while still active as a rodenticide, is frequently rejected in bait by rodents. As such, it is important to have a process capable of synthesizing the preferred N-methyl derivative without the presence of the desmethyl impurity.

The procedure currently used to synthesize bromethalin is to react the desmethyl intermediate with methylbromide under pressure. However, this process has several disadvantages. First, because of the necessity of conducting the reaction under pressure, complicated and expensive machinery is necessary which is impractical for a large scale industrial synthesis. Second, methyl bromide is an expensive reagent which is toxic to humans thus making it unacceptable for use on a large scale. Third, and most importantly, methylation with methyl bromide does not completely afford the desired N-methyl derivative and, as such, extensive purification is required to remove the desmethyl impurity.

Synthesis, No. 3, March 1980, page 215 discloses the condensation of 2-fluoronitrobenzylene with anilines and U.S. Patent No. 2,212,825 discloses that N-alkyldiphenylamines may be prepared by condensing halobenzenes with N-alkylanilines. However, it has been found that 3,5-dinitro-2-chlorobenzotrifluoride generally does not undergo such condensation. When reaction does occur, it yields only a small amount of the intended N-alkyl-2,4-dinitro-6-(trifluoromethyl)-diphenylamine, generally accompanied by the corresponding desalkyldiphenylamine; and in some instances the reaction yields only the desalkyldiphenylamine.

It has now been discovered that condensation can be effected without the problems associated with other processes as noted above including the loss of the methyl group. The present invention provides a process for preparing bromethalin intermediate N-methyl-2,4-dinitro-N-phenyl-6-(trifluoromethyl)-benzenamine by fluorine displacement of chlorine from 2-chloro-3,5-dinitrobenzotrifluoride *in situ* followed by nucleophilic displacement with N-methylaniline in the presence of N,N-dimethylformamide and pyridine.

The present invention provides a process for preparing N-methyl-2,4-dinitro-N-phenyl-6-(trifluoromethyl)benzenamine of the formula

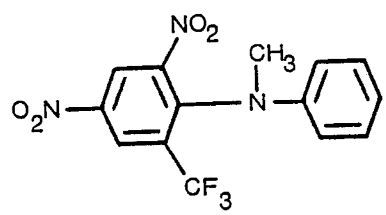

comprising the following steps carried out in N,N-dimethylformamide and pyridine under anhydrous conditions:

A. treating 2-chloro-3,5-dinitrobenzotrifluoride of the formula

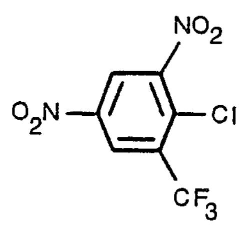

with a fluorinating reagent to provide 2-fluoro-3,5-dinitrobenzotrifluoride of the formula

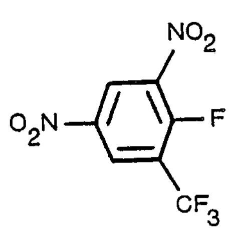

; and

B. reacting 2-fluoro-3,5-dinitrobenzotrifluoride with N-methylaniline of the formula

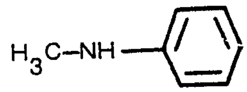

Temperatures shall be provided herein in degrees Celsius. Quantities shall be provided in weight units, except for liquids, which are set forth in volume units.

The present process is generally known as disclosed in Spanish Patent No. 315,846 without pyridine. However, the advantage of the present process lies in the use of pyridine in the process as both an acid scavenger as well as a promoter of the fluorination reaction. The use of pyridine in the present process allows for the synthesis of N-methyl-2,4-dinitro-N-phenyl-6-(trifluoromethyl)benzenamine in high yield and in pure form on a manner suitable for large scale industrial synthesis. More importantly, the present process allows for the synthesis of the N-methyldiphenylamine free from the desmethyl impurity in a reliable and consistent fashion.

The present process is conducted under anhydrous conditions. When acting in accordance with the present process, the synthesis of the desired compound may be hampered by the presence of small amounts of water in the reaction mixture since the fluorinating reagent, in particular, will consume substantially all available water before it reacts with any other compound. It is therefore preferred to use dry starting materials and solvent in the present process. Further, the operator may deem it advisable to conduct the reaction in the presence of an inert gas such as nitrogen or argon. Also, very careful drying of the equipment and purging of the reactor with inert gas are clearly advisable.

As noted above, the present process is carried out in the presence of pyridine. The quantity of pyridine employed in the present process will typically be at least approximately 1.0 molar equivalent of pyridine for each molar equivalent of 2-chloro-3,5-dinitrobenzotrifluoride. Less than 1.0 molar equivalent of pyridine may be employed when employing an excess amount of N-methylaniline, but this is less preferred due to the added cost of this expensive reagent. Preferably an excess amount of pyridine is employed in the present process, for example from about 1.1 to about 2.0 molar equivalents of pyridine for each molar equivalent of 2-chloro-3,5-dinitrobenzotrifluoride. The pyridine surprisingly and unexpectedly acts as a catalyst to accelerate the conversion of 3,5-dinitro-2-chlorobenzotrifluoride to the fluoride intermediate in the first step of the present process.

The present process is conducted in the presence of a suitable fluorinating reagent. The source of the fluoride ion is not critical. This fluorinating reagent may be chosen from among any of the alkali metal fluorides such as lithium fluoride, sodium fluoride, rubidium fluoride, cesium fluoride and especially potassium fluoride, as well as the alkaline earth metal fluorides such as calcium fluoride and magnesium fluoride. Other representative fluorinating reagents include the copper fluorides, ferrous fluoride and ferric fluoride. Of these, potassium fluoride is the preferred fluorinating reagent because of the favorable solubility of the potassium ion in the solvent system employed in the present process and the relative low cost of the reagent.

As noted above, the fluorinating reagent is generally hydroscopic in nature. It has been determined that it is preferred to thoroughly dry the reagent prior to its use in the present process. This is typically carried out by heating the reagent at a temperature in the range of about 60°C to about 150°C, at either standard or reduced pressure, for about 1 to 5 hours. The dried fluorinating reagent is then preferably combined with the other reaction ingredients while hot in order to minimize contamination by water.

Typically, a quantity of fluorinating reagent in the range of about 1.0 molar equivalent to about 4.0 molar equivalents or more for each molar equivalent of 2-chloro-3,5-dinitrobenzotrifluoride is employed in the present process. It is preferable to use an excess of fluorinating reagent in the present process, and this is not harmful to the reaction itself. Because of its sensitivity to water, it is typical to add an appropriate excess of fluorinating reagent to account for the water present in the reaction mixture.

The present process is carried out in the presence of N,N-dimethylformamide (DMF). The concentration of the reactants in N,N-dimethylformamide is not critical, but it is preferred to employ a sufficient amount of solvent to keep the reactants in solution, or a slight excess. Large volumes of solvent are not necessary or desirable in the present process.

The ensuing 2-fluoro-3,5-dinitrobenzotrifluoride intermediate can be isolated before utilization in the present process. However, the intermediate is conveniently and preferably prepared *in situ* and used without isolation in the second step of the present process.

The second step of the present process comprises reacting the 2-fluoro-3,5-dinitrobenzotrifluoride thus prepared with N-methylaniline in N,N-dimethylformamide and pyridine to provide N-methyl-2,4-dinitro-N-phenyl-6-(trifluoromethyl)benzenamine. This step of the process consumes the reactants in about equimolar amounts, and, because of the presence of pyridine, excess of N-methylaniline is not necessary as a hydrogen fluoride acceptor. This represents a significant cost savings when employing the present process. A slight excess of N-methylaniline is preferably employed in the present process, however, to account for impurities associated with this compound. Typically, 1.1 molar equivalents of N-methylaniline are employed for each molar equivalent of 2-chloro-3,5-dinitrobenzotrifluoride.

In the present process pyridine acts as a hydrogen fluoride acceptor. The quantity of pyridine

employed should be sufficient to neutralize the acid produced herein, but excess amounts have not been found to be deleterious to the process. Previously, excess N-methylaniline was employed as the acid scavenger, but this has the disadvantage of being expensive, especially on a large scale.

The present process is substantially complete after about 1 to about 24 hours when conducted at a temperature in the range of about 70°C to about 100°C, more specifically for about 3 to about 4 hours at a temperature of about 85°C to about 90°C. Yet another advantage of the present process it that it may be conducted at a temperature wherein demethylation will not occur.

The product of the present process is readily isolated by standard procedures. Typically, following cooling of the reaction mixture, the mixture is purified with infusorial earth, if necessary, and combined with toluene. The filtrate is then concentrated and the residue solubilized with a suitable water immiscible organic solvent such as toluene. The organic phase may then be washed with dilute acid, dilute base and water. The organic phase is evaporated, typically under vacuum, and the residue thus isolated is suitable for use as is for further synthetic modifications. If desired, the compound may be further purified by standard procedures such as crystallization from common solvents or column chromatography over solid supports such as silica gel or alumina.

The compound prepared by the present process is preferably used as an intermediate in the synthesis of N-methyldiphenylamines which are useful as rodenticides. *See* U.S. Patent No. 4,187,318, herein incorporated by reference, for a discussion of the synthesis of the desired N-methyldiphenylamines from the product of the present process, and their use as rodenticides.

The preferred rodenticide prepared from the product of the present process is 2,4,6-tribromo-N-methyl-2',4'-dinitro-6'-trifluoromethyldiphenylamine, generically known as bromethalin. Bromethalin may be readily synthesized from N-methyl-2,4-dinitro-N-phenyl-6-(trifluoromethyl)benzenamine prepared by the present process by bromination with elemental bromine in a basic medium, as well as other bromination reagents such as N-bromosuccinimide and dibromoisocyanuric acid.

The compounds employed as starting materials in the present process are known and readily prepared by prior art processes. For example, N-methylaniline is commercially available from a variety of sources such as Aldrich Chemical Company, Milwaukee, Wisconsin. It is important that the N-methylaniline employed herein have little or no aniline impurity associated with it. The compound 2-chloro-3,5-dinitrobenzotrifluoride may be conveniently prepared by treating commercially available 2-chlorobenzotrifluoride with nitric acid under standard nitration conditions.

The following Examples illustrate specific aspects of the process of the present invention. The Examples are not intended to be limiting to the scope of the invention in any respect and should not be so construed. In each Example, the desired compound which is synthesized is N-methyl-2,4-dinitro-N-phenyl-6-(trifluoromethyl)benzenamine. Further, the identity of the desired compound was verified by gas chromatography by comparing the retention times of a sample of the desired compound with a sample of an authentic reference standard.

## Example 1

To a 500 ml round bottom flask purged with nitrogen was added 27.0 g (0.1 mol) of 2-chloro-3,5-dinitrobenzotrifluoride, 12.0 g (0.11 mol) of N-methylaniline, 30.0 g (0.52 mol) of potassium fluoride and 100 ml of DMF at about 20°C. The reaction mixture was warmed to about 85°C and held at about 85°C to about 90°C for about one hour. To the mixture was added a solution of 9 ml of pyridine and 25 ml of DMF dropwise over one hour. The mixture was stirred for one hour at 85°—90°C and cooled overnight. To the mixture was added 50 ml of toluene and the resulting mixture was filtered. The filtrate was concentrated under vacuum and the resulting residue was solubilized with 100 ml of toluene. The resulting organic solution was washed once with 100 ml of water, three times with 100 ml portions of dilute hydrochloric acid, three times with 100 ml portions of 3% sodium hydroxide and two times with 100 ml of water. The organic phase was concentrated under vacuum to dryness to provide 27.0 g of 98.0% pure N-methyl-2,4-dinitro-N-phenyl-6-(trifluoromethyl)benzenamine as an oil. Yield 79.2%.

## Example 2

A dry 500 ml round bottom flask was purged with nitrogen and charged with 100 ml of DMF, 27.0 g (0.1 mol) of 2-chloro-3,5-dinitrobenzotrifluoride and 16.4 g (0.15 mol) of N-methylaniline at room temperature. To this mixture was added 30.0 g (0.52 mol) of potassium fluoride, and the resulting mixture was warmed to about 85°C over a period of about 15 minutes. The mixture was held at about 85°—90°C for about two hours and a solution of 5 ml of pyridine and 25 ml of DMF was added dropwise over two hours. The mixture was stirred at 85°—90°C for about two hours and 5.0 g of Celite and 50 ml of toluene was added thereto. The mixture was cooled to 50°C, filtered and the cake was washed with two 25 ml portions of toluene. The filtrate was evaporated under vacuum and the residue was solubilized with 100 ml of toluene. The resulting organic phase was washed three times with 100 ml portions of dilute hydrochloric acid, three times with 100 ml portions of 5% sodium hydroxide and one time with 100 ml of water. The organic phase was evaporated under vacuum to provide 26.4 g of 97.8% pure N-methyl-2,4-dinitro-N-phenyl-6-(trifluoromethyl)benzenamine as an oil. Yield 79.2%.

Example 3

A solution of 27.0 g (0.1 mol) of 96.7% pure 2-chloro-3,5-dinitrobenzotrifluoride in 50 ml of toluene having a temperature of about 30°C was extracted twice with 50 ml portions of 20% aqueous sodium chloride solution. The organic phase was transferred to a 500 ml round bottom flask and 50 ml of toluene was added thereto. The solution was concentrated under vacuum at a temperature of about 100°C to provide an oil. Approximately 100 ml of toluene was added to the residue and the solution was again concentrated under vacuum at about 100°C. To the dried residue was added a slurry of 30.0 g (0.52 mol) of potassium fluoride which had been previously dried in an oven, 100 ml of DMF and 12.0 g (0.11 mol) of N-methylaniline. The mixture was warmed to about 85°—90°C and held at that temperature for about 60 minutes. A gas chromatograph of the reaction mixture indicated the presence of 45.2% of the desired product. To the mixture was added a solution of 9 ml of pyridine and 25 ml of DMF dropwise over a period of about 60 minutes at 85°—90°C. A gas chromatograph of the mixture indicated the presence of 86.7% of the desired product. The mixture was stirred for an additional one hour at 85°—90°C and a gas chromatograph indicated the presence of 96.4% of the desired product. The mixture was stirred for an additional 30 minutes and 125 ml of toluene and 200 ml of water was added to the mixture which was cooled to 50°C. The organic phase was separated and washed once with 100 ml of water, three times with 100 ml portions of 3% sodium hydroxide and twice with 100 ml portions of water. The organic phase was evaporated under vacuum at about 80°C to provide 29.0 g of 97.5% pure N-methyl-2,4-dinitro-N-phenyl-6-(trifluoromethyl)benzenamine as an oil. Yield 85.0%.

Example 4

A solution of 27.0 g (0.1 mol) of 2-chloro-3,5-dinitrobenzotrifluoride in 100 ml of toluene at 30°C was extracted twice with 50 ml portions of 20% aqueous sodium chloride. The organic phase was evaporated at 95°—100°C to provide an oil. The resulting oil was stored under nitrogen for one hour and warmed to approximately 60°C. To the oil was added 15.0 g (0.26 mol) of potassium fluoride which was oven dried at 120°C. To this mixture was added 80 ml of DMF and 12.0 g (0.11 mol) of N-methylaniline. The resulting mixture was warmed to about 85°C and held at that temperature for approximately 30 minutes. To this mixture was added a solution of 9 ml of pyridine and 20 ml of DMF over 90 minutes while maintaining the temperature of the reaction mixture at about 85°—90°C. A gas chromatograph of the reaction mixture indicated the presence of 75% of the desired product. The mixture was stirred for an additional 90 minutes at 85°—90°C and a gas chromatograph indicated the presence of 96.8% product. The mixture was stirred for an additional 30 minutes at 85°—90°C and 100 ml of toluene and 100 ml of water were added thereto. The organic phase was separated and washed one time with 100 ml of water, 100 ml of dilute hydrochloric acid, 100 ml of 5% sodium hydroxide and 100 ml of water. A gas chromatograph of the mixture indicated the presence of 98.3% product. The organic phase was concentrated under vacuum to dryness at 90°C for 45 minutes to provide 29.02 g of 98.5% pure N-methyl-2,4-dinitro-N-phenyl-6-(trifluoromethyl)benzenamine as an oil. Yield 85.1%.

Example 5

A solution of 27.0 g (0.1 mol) of 2-chloro-3,5-dinitrobenzotrifluoride in 100 ml of toluene was washed with 50 ml of 20% aqueous sodium chloride solution. The organic layer was transferred to a 500 ml round bottom flask and evaporated under vacuum at 95°—100°C to provide an oily residue. To this residue was added 15.0 g (0.26 mol) of potassium fluoride which had been oven dried at 120°C for one hour. Next, 80 ml of DMF and 12.0 g (0.11 mol) of N-methylaniline was added to the reaction mixture. The mixture was warmed to 85°—90°C and held at that temperature for about 30 minutes. To this mixture was added a solution of 9 ml of pyridine and 20 ml of DMF over 90 minutes at about 85°—90°C. The mixture was stirred at that temperature for about 3 hours and a gas chromatograph of the solution indicated the presence of 97.9% of the desired product. The reaction mixture was quenched with 125 ml of toluene and 100 ml of water. The mixture was cooled to about 35°C and the organic phase was separated. The organic layer was washed with each of 100 ml of water, 100 ml of dilute hydrochloric acid, 100 ml of 10% sodium hydroxide and two 100 ml portions of water. The organic layer was concentrated under vacuum to provide 31.0 g of 98.5% pure N-methyl-2,4-dinitro-N-phenyl-6-(trifluoromethyl)benzenamine as an oil. Yield 90.9%.

Example 6

A solution of 425 ml of toluene and 115.0 g (0.43 mol) of 2-chloro-3,5-dinitrobenzotrifluoride was concentrated under vacuum at about 95°C. To the residue was added 62.0 g (1.07 mol) of potassium fluoride which had been dried at 125°C for 12 hours, 340 ml of DMF and 51.0 g (0.48 mol) of N-methylaniline. The mixture was heated at about 85°—90°C for about 30 minutes and a solution of 38.5 ml of pyridine and 85 ml of DMF was added dropwise to the mixture over 90 minutes. The mixture was stirred at about 85°—90°C for about 3 hours, and 525 ml of toluene and 425 ml of water was added. The organic phase was separated, washed with 525 ml of water, and allowed to stand overnight. The organic phase was then washed with 425 ml of dilute hydrochloric acid, 425 ml of dilute 50% aqueous sodium hydroxide (10/1, v/v), and twice with 450 ml portions of water. The organic layer was transferred to a 1 l round bottom flask and the volatiles were evaporated under reduced pressure at about 50°C. To the mixture was added 100 ml of n-butanol which was evaporated under vacuum. Next, 385 ml of 3A ethanol was added and the mixture

was warmed to 60°C. To this mixture was added a solution of 14 ml of water and 9.4 g of 50% potassium hydroxide over 5 minutes. The mixture was cooled to about 45°C and stirred at 25°—45°C for about 45 minutes. The mixture was then cooled to about −10°C and held at that temperature for 30 minutes. The precipitated solid was collected by vacuum filtration and washed twice with 50 ml portions of ethanol/water (9/1, v/v) to provide 110.0 g of N-methyl-2,4-dinitro-N-phenyl-6-(trifluoromethyl)benzenamine. The material weighed 94.1 g following drying and had a purity of 99+%. Yield 64.2%. mp = 85°—88°C.

Example 7

A solution of 115.0 g (0.43 mol) of 2-chloro-3,5-dinitrobenzotrifluoride in 425 ml of toluene was concentrated under vacuum at about 100°C. To the residue was added 62.0 g (1.07 mol) of hot potassium fluoride dried at 125°C, 340 ml of DMF and 51.0 g (0.48 mol) of N-methylaniline. The mixture was heated at about 85°—90°C for about 30 minutes and a solution of 39 ml of pyridine and 85 ml of DMF was added dropwise to the reaction mixture for 90 minutes while maintaining the temperature of the mixture between about 85°C and about 90°C. The mixture was stirred at about 85°—90°C for about 3 hours and the mixture was allowed to cool at room temperature overnight. To this mixture was added 425 ml of water and 525 ml of toluene at room temperature and the mxiture was stirred for 10 minutes. The organic phase was separated and washed with 400 ml of dilute hydrochloric acid, 400 ml of dilute sodium hydroxide (9/1, v/v) and twice with 400 ml portions 5% aqueous sodium chloride. A gas chromatograph of the organic phase indicated the presence of 99.0% of the desired N-methyl-2,4-dinitro-N-phenyl-6-(trifluoromethyl)-benzenamine. The toluene was evaporated under vacuum at about 50°C and 100 ml of n-butanol was added to the residue. The solvent was concentrated under vacuum and 380 ml of 3A ethanol was added thereto. The mixture was heated to about 65°C and 19 ml of water was added dropwise to the mixture. The mixture was allowed to cool for 60 minutes to about 30°C and then cooled with an external ice/methanol bath to about −12°C at which temperature the mixture was held for 30 minutes. The precipitated solid was collected by vacuum filtration and washed twice with 50 ml portions of ethanol/water (9/1, v/v) at about −10°C to provide 132.3 g of N-methyl-2,4-dinitro-N-phenyl-6-(trifluoromethyl)benzenamine following air drying at 45°—55°C. Yield 90.2%. Purity 99.98%. mp = 85°—89°C.

Example 8

A solution of 123.3 g (0.46 mol) of 2-chloro-3,5-dinitrobenzotrifluoride in 300 ml of toluene was concentrated under vacuum at about 95°C. The resulting hot residue was combined with 62.0 g (1.07 mol) of hot potassium fluoride which had been dried at about 90°C. To this mixture was added 300 ml of DMF and 51.0 g (0.48 mol) of N-methylaniline. The resulting mixture was heated at about 85°—90°C for about 30 minutes and 39 ml of pyridine was added dropwise to the reaction mixture for 90 minutes. The mixture was stirred at 85°—90°C for about 3 hours and cooled for 30 minutes to about 50°C. To the mixture was added 250 ml of toluene and 250 ml of water and the mixture was stirred for 10 minutes. The mixture was transferred to a separatory funnel and the reaction flask was rinsed with 50 ml of water and 50 ml of toluene. The layers were allowed to separate and a gas chromatograph of the organic phase indicated the presence of 98.5% of the desired N-methyl-2,4-dinitro-N-phenyl-6-(trifluoromethyl)benzenamine. The organic layer was separated and washed with 250 ml of dilute hydrochloric acid, 250 ml of dilute sodium hydroxide, 250 ml of 5% aqueous sodium chloride and 250 ml of 10% aqueous sodium chloride. The organic phase was concentrated under vacuum at about 50°C to about 65°C and 100 ml of n-butanol was added to the residue. This solution was concentrated under vacuum at around 85°C and the residue was dissolved in 300 ml of 3A ethanol. The resulting solution was refluxed in order to dissolve the solid matter and 15 ml of water was added. The resulting mixture was refluxed, and cooled for 75 minutes at room temperature and 45 minutes at −15°C. The precipitated solid was collected by vacuum filtration and washed twice with 50 ml portions of ethanol/water (9/1, v/v). The resulting solid was air dried at 50°C to provide 133.32 g of 99.5% pure N-methyl-2,4-dinitro-N-phenyl-6-(trifluoromethyl)benzenamine. mp = 86°—89°C. Yield 85%.

Example 9

A solution of 275 ml of toluene and 121.7 g (0.45 mol) of 2-chloro-3,5-dinitrobenzotrifluoride was distilled at atmosphereic pressure at a temperature of about 115°—125°C to provide 125 ml of toluene. The remaining solution was concentrated at about 95°—100°C. To this hot residue was added 62.0 g (1.07 mol) of hot potassium fluoride which had been previously dried in an oven and 51.0 g (0.48 mol) of N-methylaniline and 300 ml of DMF. The resulting mixture was heated for 30 minutes at 85°—90°C and 39 ml of pyridine was added to the mixture rapidly over a period of about 2 minutes. The mixture was heated at 85°—90°C for about 4 hours and a gas chromatograph of the reaction mixture indicated the presence of 81.1% of the desired product. The mixture was allowed to cool and stir overnight. The mixture was charged with 250 ml of toluene and 325 ml of water and stirred for 15 minutes at about 38°C. The mixture was transferred to a separatory funnel and the reaction flask was rinsed with 50 ml of toluene. The organic phase was separated and washed with 250 ml of dilute hydrochloric acid, 250 ml of dilute sodium hydroxide, and twice with 250 ml portions of 10% aqueous sodium chloride. The organic phase was transferred to a 500 ml round bottom flask and a gas chromatograph indicated the presence of 98.3%

product. The solution was concentrated under vacuum at about 60°C and 50 ml of n-butanol was added to the residue. The mixture was concentrated under vacuum and 300 ml of 3A ethanol was added to the residue. The mixture was heated to 80°C and a solution of 0.1 g of 50% potassium hydroxide in 15 ml of water was added to the mixture over a period of about 2 minutes. The mixture was air cooled to about 30°C and then to about −15°C at which temperature it was held for 30 minutes. The precipitated solid was collected by vacuum filtration and washed twice with 50 ml of ethanol/water (9/1, v/v) at −10°C. The resulting solid was dried at 50°—55°C to provide 128.5 g of 99.5% pure N-methyl-2,4-dinitro-N-phenyl-6-(trifluoromethyl)benzenamine. Yield 83.7%. mp = 86°—89°C.

Example 10

A solution of 121.7 g (0.45 mol) of 2-chloro-3,5-dinitrobenzotrifluoride in 275 ml of toluene was distilled under atmospheric pressure at 130°C, and then vacuum distilled at 90°—100°C to dryness. To the residue was added 62.0 g (1.07 mol) of hot oven dried potassium fluoride, 300 ml of DMF and 51.0 g (0.48 mol) of N-methylaniline. The resulting mixture was heated at about 85°—90°C for 30 minutes and 39 ml of pyridine was added in one portion. The resulting mixture was heated at about 85°—90°C for 4 hours and a gas chromatograph of the resulting mixture indicated the presence of 98.3% of the desired product N-methyl-2,4-dinitro-N-phenyl-6-(trifluoromethyl)benzenamine. The mixture was cooled to about 30°C and quenched with 300 ml of toluene and 325 ml of water. The layers were allowed to separate overnight and the organic phase was separated and washed with 250 ml of dilute hydrochloric acid, 250 ml of dilute sodium hydroxide and twice with 250 ml portions of 10% aqueous sodium chloride. The organic phase was transferred to a 500 ml round bottom flask and the toluene was concentratede under vacuum at 65°C. The residue was dissolved in 50 ml of n-butanol and the solution was concentrated under vacuum at 80°C. The mixture was cooled at room temperature for 2 hours, cooled to about 0°C for 45 minutes and finally cooled at −10°C for 20 minutes. The precipitated solid was collected by vacuum filtration and washed twice with 50 ml portions of ethanol/water (9/1, v/v) at −10°C. The solid was dried at 50°C overnight to provide 129.3 g of 99.6% pure N-methyl-2,4-dinitro-N-phenyl-6-(trifluoromethyl)benzenamine. Yield 84.3%. mp = 86°—89°C.

**Claim**

A process for preparing N-methyl-2,4-dinitro-N-phenyl-6-(trifluoromethyl)benzenamine of the formula

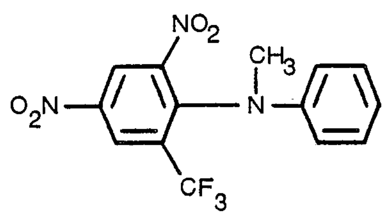

comprising the following steps carried out in N,N-dimethylformamide and pyridine under anhydrous conditions;

A. treating 2-chloro-3,5-dinitrobenzotrifluoride of the formula

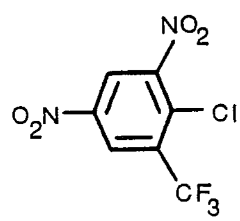

with a fluorinating reagent to provide 2-fluoro-3,5-dinitrobenzotrifluoride of the formula

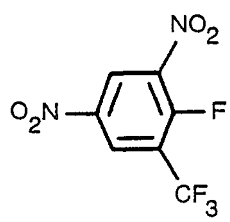

: and

B. reacting 2-fluoro-3,5-dinitrobenzotrifluoride with N-methylaniline of the formula

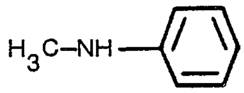

**Patentanspruch**

Verfahren zur Herstellung von N-Methyl-2,4-dinitro-N-phenyl-6-(trifluormethyl)benzolamin der Formel

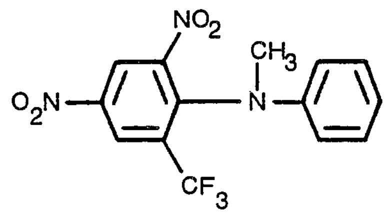

dadurch gekennzeichnet, daß die folgenden Stufen in N,N-Dimethylformamid und Pyridin unter wasserfreien Bedingungen durchgeführt werden:

A) Behandlung von 2-Chlor-3,5-dinitrobenzotrifluorid der Formel

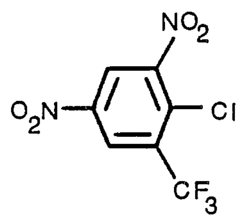

mit einem Fluorierungsmittel unter Bildung von 2-Fluor-3,5-dinitrobenzotrifluorid der Formel

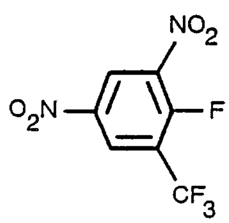

: und

und

B) Umsetzung von 2-Fluor-3,5-dinitrobenzotrifluorid mit N-Methylanilin der Formel

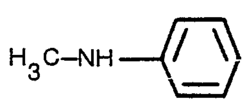

**Revendication**

Procédé pour la préparation de la N-méthyl-2,4-dinitro-N-phényl-6-(trifluoromethyl)benzènamine répondant a la formule:

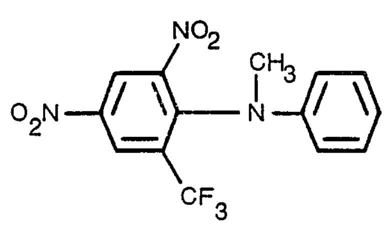

ce procédé comprenant les étapes ci-après et étant realisé dans du N,N-diméthylformamide et de la pyridine, dans des conditions anhydres:

A. traiter le 2-chloro-3,5-dinitrobenzotrifluorure de formule:

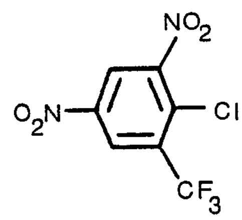

avec un agent de fluoration, pour obtenir le 2-fluoro-3,5-dinitrobenzotrifluorure de formule:

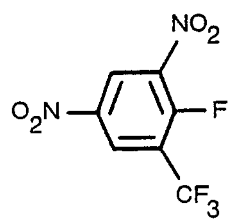

: et

B. faire réagir le 2-fluoro-3,5-dinitrobenzotrifluorure avec une N-méthylaniline de formule: